# EUROPÄISCHE PATENTSCHRIFT

(11) **EP 1 627 657 B1**
(45) Veröffentlichungstag und Bekanntmachung des Hinweises auf die Patenterteilung: **11.07.2007**
(21) Anmeldenummer: 05016865.7
(22) Anmeldetag: 03.08.2005
(51) Int. Cl.: A61M 25/00, A61M 25/06

(54) **Verfahren zum Herstellen eines Verweilkatheters sowie danach hergestellter Verweilkatheter**
Method of manufacturing an indwelling catheter and indwelling catheter produced by this method
Procédé de fabrication d'un cathéter à demeure et cathéter fabriqué selon ce procédé

(30) Priorität: 20.08.2004 DE 102004040358
(43) Veröffentlichungstag der Anmeldung: 22.02.2006
(73) Patentinhaber: Pfaff GmbH System Innovation Spritzgiesstechnik, 79183 Waldkirch (DE)
(72) Erfinder: Pfaff, Adolf, 79183 Waldkirch (DE)
(74) Vertreter: Maucher, Wolfgang

(56) Entgegenhaltungen:
- US-A- 4 846 812
- US-A- 5 156 596
- US-A1- 2001 009 988

## Beschreibung

Die Erfindung betrifft ein Verfahren zum Herstellen eines aufweitbaren Verweilkatheters mit einer Metall-Kanüle und einem die Metall-Kanüle außenseitig zumindest bereichsweise umfassenden Kunststoff-Röhrchen, welches an einem dem Einstechende der Metall-Kanüle abgewandten Befestigungsende an einer Halterung (Butterfly-Halterung) befestigt ist, wobei das Einstechende der Metall-Kanüle an dem dem Einstechende zugewandten Ende des Kunststoff-Röhrchens aus dem Kunststoff-Röhrchen herausragt sowie einen Verweilkatheter, hergestellt nach diesem Verfahren.

Derartige Verweilkatheter sollen zwei gegensätzliche Anforderungen erfüllen. Einerseits soll der Durchmesser möglichst gering sein, um ein schonendes, möglichst schmerzarmes Einführen des Katheters in die Vene zu ermöglichen und außerdem, um den Katheter auch bei dünneren zu punktierenden Venen anwenden zu können.
Andererseits soll der Strömungsquerschnitt des Katheters möglichst groß sein, um Infusionsflüssigkeiten schnell zuführen zu können, wobei auch dickflüssigere Substanzen mit hoher Infusionsgeschwindigkeit verabreichbar sein sollen.

Bekannt ist beispielsweise aus der DE 102 40 331 A1 eine Verweilkanüle, bei der das Kunststoff-Röhrchen durch eine Kunststoffkanüle gebildet ist, die mittels einer Längseinfaltung in ihrem Querschnitt verkleinert wird. Die Metall-Kanüle weist einen Längsschlitz auf, durch den die Längseinfaltung der Kunststoffkanüle ins Innere der Metall-Kanüle eingreift. Der übrige Teil der Kunststoffkanüle liegt außenseitig an der Metall-Kanüle an. In diesem querschnittreduzierten Zustand wird der Katheter gesetzt. Danach wird die Metall-Kanüle aus der Kunststoffkanüle herausgezogen, wobei sich die Längseinfaltung entfaltet und sich dabei der Querschnitt der Kunststoffkanüle vergrößert. Der größere Querschnitt wird durch eine zusätzliche Kunststoffkanüle stabilisiert.
Die Metall-Kanüle soll, wie eingangs erwähnt, einen möglichst kleinen Durchmesser aufweisen. Üblicherweise haben solche Metall-Kanülen einen Außendurchmesser von etwa 1mm bis etwa 1,5mm. Es erfordert einen hohen Aufwand, in eine solche dünne Stahlkanüle einen schmalen Längsschlitz für die Längseinfaltung der Kunststoffkanüle einzubringen. Problematisch ist dabei auch, dass die Ränder des Längsschlitzes gratfrei sein müssen, um die eingefaltete Kunststoffkanüle nicht zu beschädigen. Weiterhin ist das Einfalten der Kunststoffkanüle in den Längsschlitz problematisch und aufwendig. Schließlich erfordert auch die zusätzliche Kunststoffkanüle zum Stabilisieren der aufgefalteten Kunststoffkanüle einen zusätzlichen Aufwand.
Somit ist die Herstellung vergleichsweise aufwendig und steht einer kostengünstigen Produktion der Verweilkanüle entgegen. Dies wirkt sich insbesondere auch in Anbetracht der hohen Stückzahlen für solche Verweilkanülen besonders nachteilig aus.

Aus der US 5 156 596 A ist ein als Multilumen-Katheter ausgebildeter Verweilkatheter bekannt, der eine aufweitbare Kanüle aus Memory-Material aufweist. Der Aufbau und die Herstellung dieses Verweilkatheter in Verbindung mit einer Kanüle aus Memory-Material ist jedoch noch vergleichsweise kompliziert und aufwendig.

Es besteht daher die Aufgabe, ein Verfahren zu entwickeln, mit dem eine kostengünstige Herstellung eines aufweitbaren Verweilkatheters möglich ist.

Zur Lösung dieser Aufgabe schlägt die Erfindung insbesondere vor, dass ein aus einem Memory-Material bestehendes Kunststoff-Röhrchen in einem hinsichtlich seines Durchmessers aufgeweiteten Endzustand mit einem Ende mit der Halterung verbunden und gehalten wird, und dass anschließend der Durchmesser des Kunststoff-Röhrchens durch Recken in Längsrichtung verringert wird, bis es mit seiner Innenfläche zumindest an seinem freien Ende an der Außenfläche der Metall-Kanüle oder eines Dorns als Kanülenersatzteil anliegt, der anschließend durch eine Metall-Kanüle ersetzt wird.
Kunststoffe mit einer Memory-Charakteristik, das heißt mit einer Formgedächtnis-Charakteristik, haben sich bereits vielfach bewährt und sind vergleichsweise kostengünstig. Das Kunststoff-Röhrchen wird aus einem aufgeweiteten Zustand, der dem Endzustand entspricht, welchen es in Gebrauchsstellung in der Vene einnimmt, derartig verformt, dass das Kunststoff-Röhrchen einen verringerten Außendurchmesser aufweist, der etwa dem Außendurchmesser der Metall-Kanüle entspricht. Dadurch ist ein leichtes und schonendes Einführen auch in kleinere Venen mit Hilfe der Metall-Kanüle möglich. Der Einstechzustand des Kunststoff-Röhrchens wird durch Recken entlang der Längsachse erreicht, wobei sich das Kunststoff-Röhrchen auf der Metall-Kanüle oder einem durchmessergleichen Dorn befindet. Die Länge des Kunststoff-Röhrchens wird dabei etwas verlängert, während der Querschnitt dagegen etwas verringert wird, bis das Kunststoff-Röhrchen auf der Metall-Kanüle oder dem Dorn aufliegt.
Diese Verformung geschieht dabei unter Umgebungsbedingungen, die beispielsweise hinsichtlich der Temperatur und/oder der Feuchtigkeit nicht den Umgebungsbedingungen in Gebrauchsstellung entsprechen. Durch den Wechsel der Umgebungsbedingungen wird später dann das Formgedächtnis des Memory-Materials aktiviert, das heißt, das Kunststoff-Röhrchen nimmt bei höherer Temperatur und/oder anderer Feuchtigkeit seine ursprüngliche Form wieder ein, wobei es seinen Querschnitt aufweitet.
Wie bereits erwähnt, kann das Recken des Kunststoff-Röhrchens entweder auf der Metall-Kanüle oder auf einem durchmessergleichen Dorn erfolgen, wobei nach dem Recken der Dorn aus dem Kunststoff-Röhrchen entfernt und die Metall-Kanüle eingeführt wird. Die Verwendung des Dorns ist zweckmäßig, weil dadurch während der Verformung des Kunststoff-Röhrchens und möglicher weiterer Produktionsschritte die Metall-Kanüle nicht dem Produktionsprozess ausgesetzt ist.

Für ein schonendes Einführen des Kunststoff-Röhrchens in die Vene gemeinsam mit der Metall-Kanüle ist es zweckmäßig, wenn während oder nach dem Recken und gegebenenfalls nach einem Ablängen des Kunststoff-Röhrchens an dessen freien Endbereich ein sich an die Außenseite der Metall-Kanüle oder des Dorns (Kanülenersatzteil) annähernder Übergangskonus angeformt wird. Dieser Vorgang wird auch als Tippforming bezeichnet. Der Übergangskonus schafft einen kontinuierlichen Übergang zwischen dem Außendurchmesser der Metall-Kanüle und dem Außendurchmesser des Kunststoff-Röhrchens.

Vorteilhaft ist es, wenn das Kunststoff-Röhrchens zur Befestigung an der Halterung an seinem dem Einstechende der Metall-Kanüle abgewandten Ende auf einen Spanntrichter aufgeschoben und zusammen mit diesem in eine Aufnahmehöhlung der Halterung eingeschoben, insbesondere eingepresst wird. Der Spanntrichter weist dazu einen Außendurchmesser auf, welcher etwas größer als der Innendurchmesser des Kunststoff-Röhrchens im aufgeweiteten Endzustand ist und wird von dem dem Kunststoff-Röhrchen abgewandten Ende der Halterung in die Halterung eingeführt. Der Spanntrichter kann aus Metall oder Kunststoff bestehen. Das Kunststoff-Röhrchen ist zwischen der Wandung der Aufnahmehöhlung und dem Spanntrichter eingespannt und gehalten, sodass ein sicherer Halt des Kunststoff-Röhrchens beim Herausziehen der Metall-Kanüle aus dem Kunststoff-Röhrchen oder beim Recken und sonstigen Zug-Belastungen vorhanden ist.

Für eine besonders gute Gleitfähigkeit des Kunststoff-Röhrchens auf der Metall-Kanüle oder dem Dorn, welches wie die Metall-Kanüle vorzugsweise aus Stahl oder dergleichen Metall besteht, ist es zweckmäßig, wenn die Oberfläche der Metall-Kanüle und/oder des Dorns silikonisiert ist, das heißt einen dünnen Silikonüberzug aufweist. Auch andere, die Gleitfähigkeit erhöhende Überzüge, beispielsweise aus Teflon sind denkbar. Dadurch ist das Recken des Kunststoff-Röhrchens auf dem Dorn oder der Metall-Kanüle, das Einsetzen und/oder das Herausziehen des Dorns oder der Metall-Kanüle aus dem Kunststoff-Röhrchen vereinfacht werden.

Es besteht nach einer vorteilhaften Variante auch die Möglichkeit, dass der Verweilkatheter zumindest mit seiner Halterung (Butterfly-Halterung) sowie dem Kunststoff-Röhrchen einstückig aus einem Memory-Material hergestellt und dann das Kunststoff-Röhrchen in Längsrichtung gereckt und dabei der Durchmesser des Kunststoff-Röhrchens verringert wird. Es wird damit also ein Verweilkatheter hergestellt, der mit seiner Halterung (Butterfly-Halterung) sowie dem Kunststoff-Röhrchen einstückig aus einem Memory-Material besteht.
Dies erspart die sonst erforderliche Montagearbeit, bei der die Halterung sowie dem Kunststoff-Röhrchen miteinander verbunden werden müssen. Die insgesamt etwas höheren Materialkosten werden durch die Einsparungen bei der Herstellung mehr als kompensiert, insbesondere wenn die Herstellung durch Spritzgießen erfolgt.
Das aus einem Memory-Material bestehende Kunststoff-Röhrchen und die an einem Ende sich daran anschließende Halterung werden somit bereits bei der Herstellung miteinander verbunden.
Bei Kontakt mit Blut beziehungsweise einer Vene oder dergleichen und die dadurch verursachte Erwärmung des Kunststoff-Röhrchens erfolgt eine Aufweitung des Kunststoff-Röhrchens in den ursprünglichen Zustand, während das übrige Memorymaterial nicht aufweitet, da dieses durch Recken oder dergleichen Formveränderung nicht vorbehandelt wurde.

Besteht nur das Kunststoff-Röhrchen aus Memorymaterial, so wird dieses Kunststoff-Röhrchen vorzugsweise in einem Extrusionsverfahren hergestellt. Besteht der Verweilkatheter insgesamt aus Memorymaterial, so erfolgt eine Herstellung vorzugsweise durch Spritzgießen, insbesondere durch Mikrosystemspritzgießen.

Für den Einsatz des nach dem erfindungsgemäßen Verfahrens hergestellten Verweilkatheters insbesondere in der Humanmedizin ist es zweckmäßig, wenn der Innendurchmesser des Kunststoff-Röhrchens bei einer Temperatur von mehr als etwa 35°C um bis zu etwa 50% infolge des Memory-Effekts des Kunststoff-Röhrchen-Materials aufgeweitet wird. Diese Aufweitung des Durchmessers um etwa 50% bedeutet eine Verdopplung der Querschnittsfläche des Kunststoff-Röhrchens. Das bedeutet auch, dass durch eine vergleichsweise geringe Veränderung des Durchmessers eine dazu vergleichsweise starke Vergrößerung des Infusionsströmungsquerschnitts erreicht werden kann.

Nachstehend ist ein Ausführungsbeispiel der Erfindung anhand der Zeichnungen näher beschrieben. Es zeigt in zum Teil schematisierter Darstellung:
- Fig. 1: eine vergrößerte Längsschnittdarstellung eines nach dem erfindungsgemäßen Verfahrens hergestellten Verweilkatheters entsprechend der Schnittlinie A-A in Fig. 2,
- Fig. 2: eine Aufsicht des Verweilkatheters etwa in Originalgröße,
- Fig. 3: eine perspektivische Darstellung des Verweilkatheters in einer Zwischenmontagestellung,
- Fig. 4: eine vergrößerte Längsschnittdarstellung des Verweilkatheters mit einseitig befestigtem Kunststoff-Röhrchen in einem aufgeweiteten Anfangszustand sowie
- Fig. 5: eine vergrößerte Schnittdarstellung einer Halterung mit dem mittels eines Spanntrichters gehaltenen Kunststoff-Röhrchen.

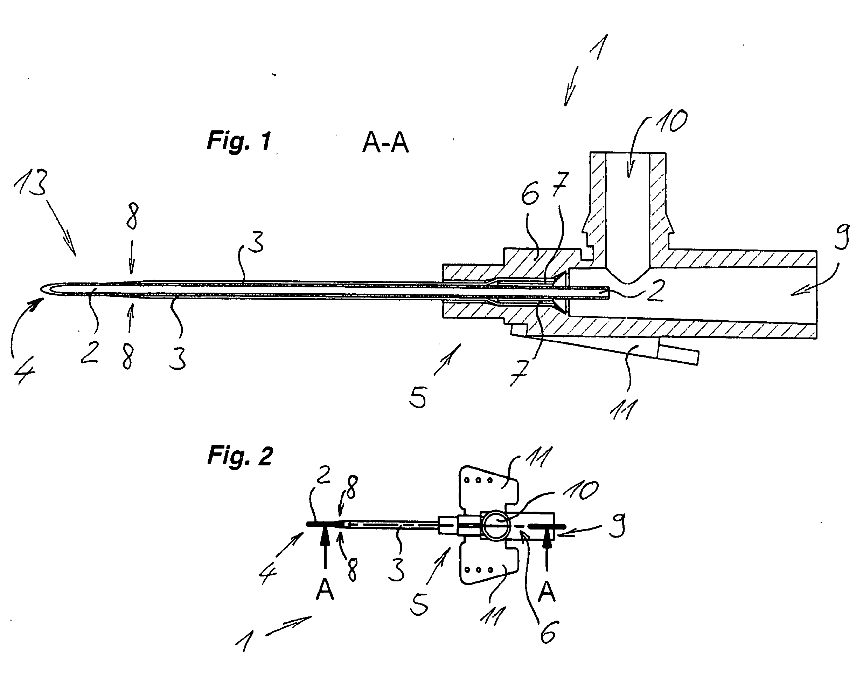

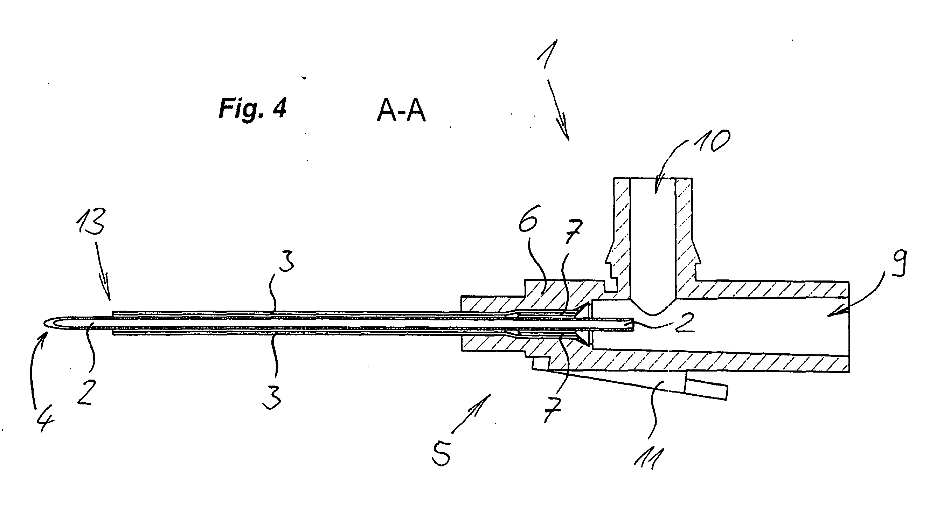

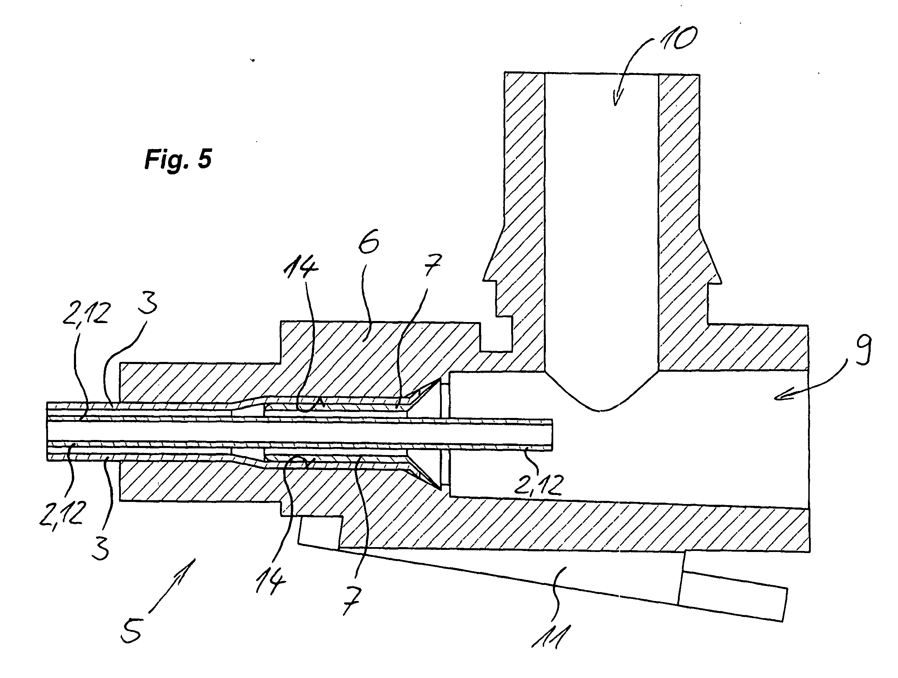

Ein im Ganzen mit 1 bezeichneter aufweitbarer Verweilkatheter weist eine Metall-Kanüle 2 und eine die Metall-Kanüle 2 bereichsweise umfassendes Kunststoff-Röhrchen 3 aus einem Memory-Material auf. Das Kunststoff-Röhrchen 3 ist, wie in den Fig. 1, 4 und 5 erkennbar, an einem dem Einstechende 4 der Metall-Kanüle 2 abgewandten Befestigungsende 5 an einer Halterung 6 (Butterfly-Halterung) befestigt. Das Kunststoff-Röhrchen 3 weist gegenüber der Metall-Kanüle 2 eine etwas geringere Länge auf, sodass das Einstechende 4 der Metall-Kanüle 2 über das freie Ende 13 des Kunststoff-Röhrchens 3 übersteht.

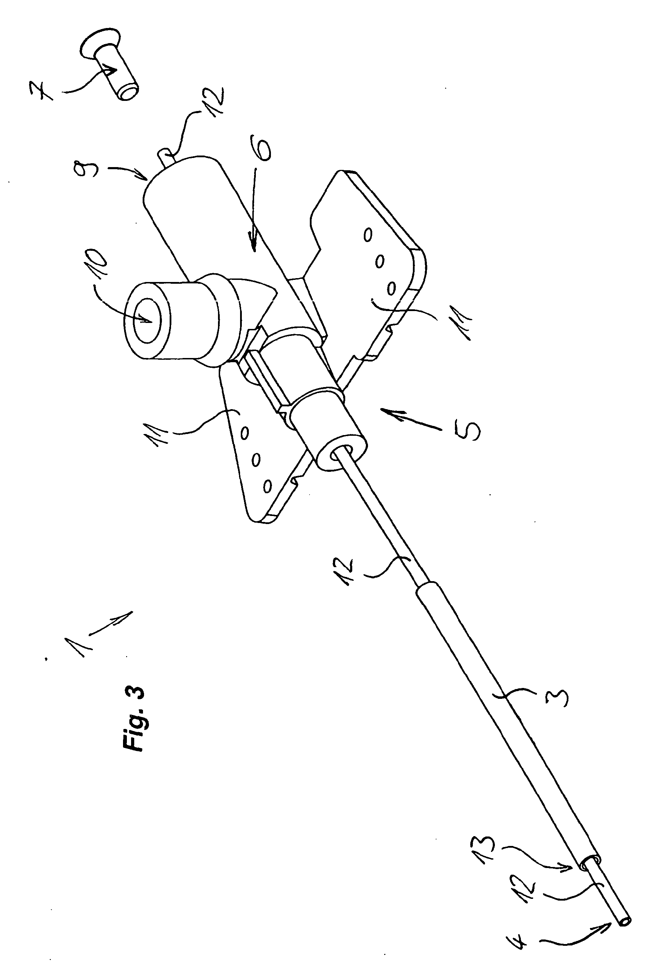

Fig. 3 zeigt den Verweilkatheter 1 in einer Zwischenmontagestellung vor der Befestigung des Kunststoff-Röhrchens 3 an der Halterung 6. Die Metall-Kanüle 2 ist in diesem Verfahrensschritt durch einen Dorn 12 mit gleichem Durchmesser wie die Metall-Kanüle 2 ersetzt. Zur Befestigung des Kunststoff-Röhrchens 3 wird in die Halterung 6 ein Spanntrichter 7 von dem rückseitigen Ende der Halterung 6 her eingeführt. Das Kunststoff-Röhrchen 3 wird auf den Spanntrichter 7 aufgeschoben und dabei am Endabschnitt etwas aufgeweitet Zustands aufgeweitet. Zusammen mit dem Kunststoff-Röhrchen 3 wird der Spanntrichter 7 dann in eine Aufnahmehöhlung 14 der Halterung 6 eingeführt und dort vorzugsweise durch Presssitz gehalten (Fig. 1, 4, 5).

Nach dem Befestigen des Kunststoff-Röhrchens 3 an der Halterung 6 wird das Kunststoff-Röhrchen 3 in Längsrichtung auf dem Dorn 12 gereckt, bis das Kunststoff-Röhrchen 3 mit seiner Innenfläche an der Außenfläche des Dorns 12 anliegt. Nach diesem Reckvorgang kann das Kunststoff-Röhrchen 3 bedarfsweise auf eine gewünschte Länge abgelängt werden. Bei oder nach dem Reckvorgang wird an das Kunststoff-Röhrchen 3 an dem freien Ende 13 des Kunststoff-Röhrchens 3 ein Übergangskonus 8 angeformt (tippforming). Anschließend wird das Dorn 12 entfernt und die Metall-Kanüle 2 in die Halterung 6 und das Kunststoff-Röhrchen 3 eingeschoben.

Das Recken und das Anformen des Übergangskonus 8 kann anstatt auf dem Dorn 12 auch auf der Metall-Kanüle 2 vorgenommen werden. Den fertigen Zustand des Verweilkatheters 1 zeigen die Fig. 1 und 2.

In den Figuren ist an der Halterung 6 ein Nebeneinlass 10 erkennbar, durch den zusätzlich zu der Infusionsflüssigkeit, die durch einen Haupteinlass 9 des Verweilkatheters 1 zugeführt wird, zum Beispiel Medikamente zugeführt werden können.

Der Nebeneinlass 10 mündet in den Strömungskanal des Haupteinlasses 9, sodass sich dort beide Flüssigkeiten vor dem Eintritt in das Kunststoff-Röhrchen 3 vermischen können und gemeinsam durch das Kunststoff-Röhrchen 3 in die Vene geleitet werden können.

An der auch als "Butterfly" bezeichneten Halterung 6 aus Kunststoff sind, wie in den Fig. 2 und 3 erkennbar, Flügel 11 angeordnet, die der Fixierung des Verweilkatheters 1 am Körper dienen.

## Patentansprüche

1. Verfahren zum Herstellen eines aufweitbaren Verweilkatheters (1) mit einer Metall-Kanüle (2) und einem die Metall-Kanüle (2) außenseitig zumindest bereichsweise umfassenden Kunststoff-Röhrchen (3), welches an einem dem Einstechende (4) der Metall-Kanüle (2) abgewandten Befestigungsende (5) an einer Halterung (6) befestigt ist, wobei das Einstechende (4) der Metall-Kanüle (2) an dem dem Einstechende (4) zugewandten Ende des Kunststoff-Röhrchens (3) aus dem Kunststoff-Röhrchen (3) herausragt, **dadurch gekennzeichnet, dass** ein aus einem Memory-Material bestehendes Kunststoff-Röhrchen (3) in einem hinsichtlich seines Durchmessers ausgeweiteten Endzustand mit einem Ende (5) mit der Halterung (6) verbunden und gehalten wird, und dass anschließend der Durchmesser des Kunststoff-Röhrchens (3) durch Recken in Längsrichtung verringert wird, bis es mit seiner Innenfläche zumindest an seinem freien Ende an der Außenfläche der Metall-Kanüle (2) oder eines Dorns (12) als Kanülenersatzteil anliegt, der anschließend durch eine Metall-Kanüle (2) ersetzt wird.

2. verfahren nach Anspruch 1, **dadurch gekennzeichnet, dass** während oder nach dem Recken und gegebenenfalls nach einem Ablängen des Kunststoff-Röhrchens (3) an dessen freien Endbereich ein sich an die Außenseite der Metall-Kanüle (2) oder des Dorns (12) annähernder Übergangskonus (8) angeformt wird.

3. Verfahren nach Anspruch 1 oder 2, **dadurch gekennzeichnet, dass** das Kunststoff-Röhrchens (3) zur Befestigung an der Halterung (6) an seinem dem Einstechende (4) der Metall-Kanüle (2)abgewandten Ende (5) auf einen Spanntrichter (7) aufgeschoben und zusammen mit diesem in eine Aufnahmehöhlung der Halterung (6) eingeschoben, insbesondere eingepresst wird.

4. Verfahren nach Anspruch 1 oder 2, **dadurch gekennzeichnet, dass** der Verweilkatheter mit seiner Halterung (6) sowie dem Kunststoff-Röhrchen (3), einstückig aus einem Memory-Material hergestellt wird und dass das Kunststoff-Röhrchen (3) dann in Längsrichtung gereckt und dabei der Durchmesser des Kunststoff-Röhrchens (3) verringert wird.

5. Verfahren nach einem der Ansprüche 1 bis 4, **dadurch gekennzeichnet, dass** der Innendurchmesser des Kunststoff-Röhrchens (3) bei einer Temperatur von mehr als etwa 35°C um bis zu etwa 50% infolge des Memory-Effekts des Kunststoff-Röhrchen-Materials aufgeweitet wird.

6. Verfahren nach einem der Ansprüche 1 bis 3 oder 5, **dadurch gekennzeichnet, dass** das aus Memorymaterial bestehende Kunststoff-Röhrchen durch Extrudieren hergestellt wird.

7. Verfahren nach einem der Ansprüche 1, 2, 4 oder 5, **dadurch gekennzeichnet, dass** der praktisch insgesamt aus Memory-Material bestehende Verweilkatheter durch Spritzgießen, insbesondere durch Mikrosystemspritzgießen hergestellt wird.

8. Verweilkatheter mit einem Kunststoff-Röhrchen (3) aus Memory-Material mit Halterung (6) und mit einer metall-Kanüle (2) mit aus dem Kunststoff-Röhrchen (3) herausragendem Einstechende, wobei das Kunststoff-Röhrchen (3) an der Außenfläche der Metall-Kanüle anliegt, **dadurch gekennzeichnet, dass** die Halterung (6) sowie das Kunststoff-Röhrchen (3) einstückig aus einem Memory-Material und die Halterung (6) aus unrecktem Memory-Material und Kunststoff-Röhrchen (3) aus gerecktem Memory-Material bestehen.

## Claims

1. Method of producing an expandable indwelling catheter (1) having a metal cannula (2) and a plastic tube (3) externally surrounding the metal cannula at least in parts, said tube (3) being attached to a holder (6) at a fixing end (5) remote from the insertion end (4) of the metal cannula (2), wherein the insertion end (4) of the metal cannula (2) protrudes from the plastic tube (3) at the end of the plastic tube (3) nearest the insertion end (4), **characterised in that** a plastic tube (3) consisting of a memory material is attached to the holder (6) at one end and held in a final state with an expanded diameter, and **in that** the diameter of the plastic tube (3) is then reduced by stretching in the longitudinal direction, until it comes to abut, at least at its free end, with its inner surface on the outer surface of the metal cannula (2) or on the outer surface of a rod (12) as a cannula substitute, which is subsequently replaced by a metal cannula (2).

2. Method according to claim 1, **characterised in that** during or after the stretching of the plastic tube (3)and optionally after cutting it to length, a transition cone (8) approaching the outside of the metal cannula (2) or the rod (12) is formed on the free end portion of said plastic tube (3).

3. Method according to claim 1 or 2, **characterised in that** for attachment to the holder (6) the plastic tube (3) is pushed onto a clamping funnel (7) at its end (5) remote from the insertion end (4) of the metal cannula (2) and is inserted, particularly pressed, together with this funnel (7) into a receiving cavity of the holder (6).

4. Method according to claim 1 or 2, **characterised in that** the indwelling catheter with its holder (6) and the plastic tube (3) is made in one piece from memory material and **in that** the plastic tube (3) is then stretched in the longitudinal direction and the diameter of the plastic tube (3) is thereby reduced.

5. Method according to one of claims 1 to 4, **characterised in that** the internal diameter of the plastic tube (3) is expanded at a temperature of more than about 35°C by up to about 50% as a result of the memory effect of the material of the plastic tube.

6. Method according to one of claims 1 to 3 or 5, **characterised in that** the plastic tube made of memory material is produced by extrusion.

7. Method according to one of claims 1, 2, 4 or 5, **characterised in that** the indwelling catheter consisting virtually entirely of memory material is produced by injection moulding, particularly by microsystem injection moulding.

8. Indwelling catheter having a plastic tube (3) of memory material with a holder (6) and a metal cannula (2) with an insertion end protruding from the plastic tube (3), wherein the plastic tube (3) abuts on the outer surface of the metal cannula, **characterised in that** the holder (6) and the plastic tube (3) are integrally formed from a memory material and the holder (6) is made of unstretched memory material and the plastic tube (3) is made of stretched memory material.

## Revendications

1. Procédé de fabrication d'un cathéter à demeure (1) pouvant être élargi, doté d'une canule métallique (2) et d'un tube (3) en matière plastique entourant au moins par endroits la canule métallique (2) côté extérieur, ledit tube étant fixé sur un support (6) au niveau d'une extrémité de fixation (5) opposée à une extrémité de piqûre (4) de la canule métallique (2), l'extrémité de piqûre (4) de la canule métallique (2) dépassant du tube (3) en matière plastique au niveau de l'extrémité du tube (3) en matière plastique tournée vers l'extrémité de piqûre (4), **caractérisé en ce qu'**un tube (3) en matière plastique se composant d'un matériau à mémoire de forme dans un état final élargi au niveau de son diamètre est relié et maintenu par une extrémité (5) au support (6), et **en ce qu'**ensuite le diamètre du tube (3) en matière plastique est réduit par étirage dans le sens longitudinal jusqu'à ce que ledit tube avec sa surface intérieure s'appuie au moins au niveau de son extrémité libre sur la surface extérieure de la canule métallique (2) ou d'un mandrin (12) comme pièce de rechange de canule, qui est ensuite remplacé par une canule métallique (2).

2. Procédé selon la revendication 1, **caractérisé en ce que** pendant ou après l'étirage et, le cas échéant, après un tronçonnage du tube (3) en matière plastique un cône de transition (8) s'approchant de la face extérieure de la canule métallique (2) ou du mandrin (12) est formé sur la zone d'extrémité libre dudit tube.

3. Procédé selon la revendication 1 ou 2, **caractérisé en ce que** le tube (3) en matière plastique, pour la fixation sur le support (6), est poussé au niveau de son extrémité (5) opposée à l'extrémité de piqûre (4) de la canule métallique (2) sur un entonnoir de serrage (7) et, conjointement avec ledit entonnoir, il est introduit, en particulier enfoncé, dans un creux de réception du support (6).

4. Procédé selon la revendication 1 ou 2, **caractérisé en ce que** le cathéter à demeure avec son support (6) ainsi qu'avec le tube (3) en matière plastique est fabriqué d'une seule pièce à partir d'un matériau à mémoire de forme, et **en ce que** le tube (3) en matière plastique est alors étiré dans le sens longitudinal et le diamètre du tube (3) en matière plastique est ainsi réduit.

5. Procédé selon l'une quelconque des revendications 1 à 4, **caractérisé en ce que** le diamètre intérieur du tube (3) en matière plastique s'élargit à une température de plus de 35°C environ d'un pourcentage pouvant aller jusqu'à 50 % environ à la suite de l'effet mémoire de forme du matériau du tube en matière plastique.

6. Procédé selon l'une quelconque des revendications 1 à 3 ou 5, **caractérisé en ce que** le tube en matière plastique se composant du matériau à mémoire de forme est fabriqué par extrusion.

7. Procédé selon l'une quelconque des revendications 1, 2, 4 ou 5, **caractérisé en ce que** le cathéter à demeure se composant pratiquement globalement de matériau à mémoire de forme est fabriqué par moulage par injection, en particulier par moulage par injection de microsystème.

8. Cathéter à demeure muni d'un tube (3) en matière plastique se composant d'un matériau à mémoire de forme avec un support (6) et une canule métallique (2), doté d'une extrémité de piqûre dépassant du tube (3) en matière plastique, ledit tube (3) en matière plastique s'appuyant sur la surface extérieure de la canule métallique, **caractérisé en ce que** le support (6) ainsi que le tube (3) en matière plastique se composent d'un seul tenant d'un matériau à mémoire de forme, et le support (6) se compose d'un matériau à mémoire de forme non étiré et le tube (3) en matière plastique se compose d'un matériau à mémoire de forme étiré.
